(19) **Europäisches Patentamt / European Patent Office / Office européen des brevets**

(11) **EP 2 051 632 B1**

(12) **EUROPEAN PATENT SPECIFICATION**

(45) Date of publication and mention
of the grant of the patent:
**12.05.2010 Bulletin 2010/19**

(21) Application number: **07802445.2**

(22) Date of filing: **31.07.2007**

(51) Int Cl.:
*A61B 5/08* (2006.01)     *A61B 5/103* (2006.01)

(86) International application number:
**PCT/EP2007/057919**

(87) International publication number:
**WO 2008/015222 (07.02.2008 Gazette 2008/06)**

(54) **DUAL BODY PLETHYSMOGRAPHY APPARATUS AND PROCESS FOR MEASURING BLOOD FLOW BETWEEN THE THORAX AND ABDOMEN (THE TRUNK) AND THE BODY PERIPHERY**

DUALKÖRPER-PLETHYSMOGRAPHIEGERÄT UND VERFAHREN ZUR MESSUNG DES BLUTFLUSSES ZWISCHEN THORAX UND ABDOMEN (RUMPF) UND DER KÖRPERPERIPHERIE

APPAREIL DE PLÉTHYSMOGRAPHIE CORPORELLE DOUBLE ET PROCÉDÉ PERMETTANT DE MESURER LE DÉBIT SANGUIN ENTRE LE THORAX ET L'ABDOMEN (LE TRONC) ET LA PÉRIPHÉRIE DU CORPS

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **02.08.2006 IT MI20061540**

(43) Date of publication of application:
**29.04.2009 Bulletin 2009/18**

(73) Proprietor: **Politecnico di Milano**
**20133 Milano (IT)**

(72) Inventors:
• **ALIVERTI, Andrea**
**I-22100 Como (IT)**
• **DELLACA', Raffaele**
**I-22100 Como (IT)**

• **PEDOTTI, Antonio**
**I-20129 Milano (IT)**
• **MACKLEM, Peter T.**
**Lansdowne, Ontario K0E 1L0 (CA)**

(74) Representative: **Mittler, Enrico**
**Mittler & C. S.r.l.**
**Viale Lombardia, 20**
**20131 Milano (IT)**

(56) References cited:
**GB-A- 2 116 725     US-A- 3 511 237**
**US-A- 5 331 968**

• **ALIVERTI A ET AL: "Opto-electronic plethysmography" MONALDI ARCHIVES FOR CHEST DISEASES, NAPLES, IT, vol. 59, no. 1, January 2003 (2003-01), pages 12-16, XP009091654 ISSN: 1122-0643**

## Description

The present invention relates to a dual body plethysmography apparatus and process for measuring the blood flow between the trunk and the body periphery.

The measurement of the blood flow between the trunk and the body periphery (term by which the upper limbs, the lower limbs and the head are designated) is extremely important in the understanding of the physiology and physiopathology of cardiopulmonary interaction.

Changes of the pleural and abdominal pressure have important implications for cerebral blood flow, pulmonary blood volume, cardiac output and ventricular afterload. Changes in these parameters affect alveolar gas exchange and skeletal muscle perfusion particularly during physical exercise with or without limitation of expiratory flow as frequently occurs in chronic obstructive lung disease and asthma. These parameters are also affected by positive pressure mechanical ventilation, negative inspiratory pressures during obstruction of the upper respiratory tract (for instance in obstructive sleep apnoea), strongly positive expiratory pressures as occurs in Valsalva's maneuver, expulsive maneuvers, and cough.

Some of the aforesaid implications have been described in the following papers:

Aliverti A., Macklem P.T., "How and why exercise is impaired in COPD", Respiration. 68: 229:239, 2001;

Aliverti A., R. L. Dellacà, P. Lotti, S. Bertini, R. Duranti, G. Scano, J. Heyman, A. Lo Mauro, A. Pedotti, P. T. Macklem. "Influence of expiratory flow-limitation during exercise on systemic oxygen delivery in humans". European Journal of Applied Physiology, 2005;

Duke G. J. "Cardiovascular effects of mechanical ventilation". Critical Care and Resuscitation 1999; 1: 388-399;

H. Fessler, S. Permutt. "Interaction between the circulatory and ventilatory pumps". Thorax: 57, 1995;

Iandelli I, Aliverti A, Kayser B, Dellacà R, Cala SJ, Duranti R, Kelly S, Scano G, Sliwinski P, Yan S, Macklem PT, Pedotti A. "Determinants of exercise performance in normal men with externally imposed expiratory flow limitation". J Appl Physiol 92: 1943-1952, 2002;

Lloyd. C.. Mechanical cardiopulmonary interdependence. "J. Appl. Physiol.: Respirat. Environ". Exercise Physiol. 52: 333-339, 1982;

Lu, K., J.W. Clark, Jr., F.H. Ghorbel, D.L. Ware, and A. Bidani. "A human cardiopulmonary system model applied to the analysis of the Valsalva maneuver". Am J Physiol Heart Circ Physiol 281: H2661-H2679, 2001;

Miller J.D., Pegelow D.F., Jacques A. J. and Dempsey J. "Effects of augmented respiratory muscle pressure production on locomotor limbs venous return during calf contraction exercise". Articles in Pres. J Appl Physiol, 2005b;

Permutt S., A. Wise. "Mechanical interaction of respiration and circulation". Handbook of physiology: the respiratory system III: 36, 1986;

Ranieri V.M., M. Dambrosio, N. Brienza. "Intrinsic PEEP and cardiopulmonary interaction in patients with COPD and acute ventilatory failure". Eur Respir J, 9:1283-1292, 1996;

Scharf, S.M., J.A. Bianco, D.E. Tow, and R. Brown. "The effects of large negative intrathoracic pressure on left ventricular function in patients with coronary artery disease". Circulation 63: 871-875, 1981;

Scharf, S.M., R. Brown, N. Saunders, and L.H. Green. "Effects of normal and loaded spontaneous inspiration on cardiovascular function". J. Appl. Physiol.: Respirat. Environ. Exercise Physiol. 47: 582-590, 1979;

Takata, M., Wise, R. A., and Robotham, J. L. "Effects of abdominal pressure on venous return: abdominal vascular zone conditions". J. Appl. Physiol., 69(6): 1961-1972, 1990.

There are currently no devices or processes which can measure blood shifts between the trunk and the extremities..

It is known that, during every respiratory act or maneuver, the variations of the volume defined by the thoraco-abdominal wall (hereafter also indicated by the symbol $\Delta v_{cw}$, with reference to the term "chest wall") are determined by the sum of the variations of gas volume in the lung (hereafter also indicated by the symbol $\Delta VL$) and variations of blood volume contained within the trunk. ($V_b$) according to the equation

$$\Delta V_{cw} = \Delta VL + V_b.$$

There are known systems to measure $\Delta VL$, by total body plethysmographies or by the acronym WBP, "Whole Body Plethysmography", described in patent US3511237) and consisting in measuring the variations of air flow or volume in a constant pressure booth in which a subject under examination is seated; furthermore, the subject breathes through a mouthpiece which is connected to the outside; finally, there is an opening provided with a special sensor that measures the $\Delta VL$. Two embodiments of such plethysmographies are considered here:

-   volume plethysmographs: the volume of input/output air to/from the booth is measured; see Mead J. "Volume displacement body plethysmograph for respiratory measurements in human subjects". J Appl Physiol 1960; 15: 736- 740;

-   flow plethysmographs: the flow of input/output air to/from the booth is measured; such a flow is then integrated in order to obtain $\Delta VL$;

There are also known systems for the measurement of the variations of the volume of the trunk ($\Delta V_{cw}$), known as thoraco-abdominal plethysmographs or by the acronym TAP, "Thoraco-Abdominal Plethysmography".

A first example of thoraco-abdominal plethysmography is given by the opto-electronic plethysmography (also known by the acronym OEP, "Opto-Electronic Plethysmography"), described in patent ITMI001188A1 and by the papers:

Ferrigno G, Carnevali P, Aliverti A, Molteni F, Beulke G and Pedotti A. "Three-dimensional Optical Analysis of Chest Wall Motion". J Appl Physiol 77(3): 1224-1231, 1994;

Cala SJ, Kenyon C, Ferrigno G, Carnevali P, Aliverti A, Pedotti A, Macklem PT and Rochester DF. "Chest wall and lung volume estimation by optical reflectance motion analysis". J Appl Physiol 81(6): 2680-2689, 1996;

Carnevali P, Ferrigno G, Aliverti A and Pedotti A. "A new method for 3D optical analysis of chest wall motion". Technology and Health Care 4: 43-65, 1996;

Aliverti A, R. Dellacà , P. Pelosi, D. Chiumello, A. Pedotti, L. Gattinoni. "Opto-electronic plethysmography in intensive care patients". Am. J. Respir. Crit. Care Med., 161: 1546-1552, 2000;

Aliverti A, R. Dellacà, P. Pelosi, D. Chiumello, L. Gattinoni and A. Pedotti. "Compartmental analysis of breathing in the supine and prone positions by Opto-Electronic Plethysmography". Ann Biomed Eng 29: 60-70, 2001;

Aliverti A, Pedotti A. "Opto-electronic Plethysmography". Monaldi Arch Chest Dis; 59(1): 12-6, 2003.

Said opto-electronic plethysmography uses digital video cameras to film a subject under examination; reflective markers are applied on the chest and abdomen of such a subject. The markers are filmed by the video cameras, which transmit the filmed sequences to a microprocessor system, which traces the position in space of the markers, and computes the variations of the volume enclosed in the trunk ($V_{cw}$) on the basis of a preestablished geometrical model.

A second example of thoraco-abdominal plethysmography is given by the respiratory inductance plethysmography (also known by the acronym RIP), described by patents US5331968, US3731184, US4308872, US4373534, US4433693, US4452252, US4494553, US4807640, US4815473, US4817625 and US4834109.

Such a respiratory inductance plethysmography uses two separate windings of wire, stitched within an elastic band which is about 10 cm broad, positioned just under the armpits and at the level of the umbilicus. The wires of the two bands, which are equivalent to as many coils, display a self-inductance that varies according to the surface thereof, which in turn depends on the volume variations of the thoraco-abdominal compartment ($\Delta V_{cw}$); in this manner, the volume variations of the thoraco-abdominal compartment ($\Delta V_{cw}$) may be traced by traditional electronic means.

A third example of thoraco-abdominal plethysmography is described by patent GB2116725A, which discloses a thoraco-abdominal plethysmograph integrated in clothing; in this case, the measurement of the volume is obtained by the measurement of appropriate resistances.

Scientific papers in this respect are:

E. R. Post and M. Orth, "Smart Fabric, or Washable Computing", Proceedings of First International Symposium on Wearable Computers, Cambridge, MA, IEEE Computer Society, Los Alamitos, CA (1997), pp. 167-168;

Della Santa A, Mazzoldi A, De Rossi D. "Dressware: wearable hardware". Material Sci Eng 1999;C7: 31-37;

Di Rienzo M, Rizzo F, Parati G, Brambilla G, Ferratini M, Castiglioni P. MagIC System: "a New Textile-Based Wearable Device for Biological Signal Monitoring. Applicability in Daily Life and Clinical Setting". In Proc. IEEE EMB Conference 2005, Shanghai. IEEE Press.

It is the object of the present invention to obtain an apparatus that measures the blood flow between the trunk and the extremities in a non-invasive manner.

According to the invention, such an object is achieved by means of an apparatus for measuring the blood flow between the trunk and the extremities ($V_b$), which comprises a whole body plethysmograph for measuring $\Delta VL$, a thoraco-abdominal plethysmograph for measuring the volume variations of the trunk ($\Delta V_{cw}$) and processing means to analyze said $\Delta VL$ with said $\Delta V_{cw}$ in order to measure the blood flow between the trunk and the extremities $V_b$, as the difference between $\Delta V_{cw}$ and $\Delta VL$:

$$V_b = \Delta V_{cw} - \Delta VL .$$

The invention also relates to a process for measuring the blood flow between the trunk and the body periphery ($V_b$), comprising:

- a measurement of the volume variations of the trunk ($\Delta V_{cw}$) by means of thoraco-abdominal plethysmography;

- a measurement of the variations of gas volume in the lungs ($\Delta VL$) by whole body plethysmography concurrent to the above said thoraco-abdominal plethysmography;

- a processing of the volume variations of the trunk ($\Delta V_{cw}$) and of the volume variations of the lungs so as to measure the blood flow between the trunk and the extremities ($V_b$).

By means of an apparatus or a process according to the invention, the blood flow from the trunk to the extremities may be derived from the equation

$$V_b = \Delta V_{cw} - \Delta VL .$$

Furthermore, by knowing the instantaneous values of the volume variations of the trunk and the values of the variations of gas volume in the lungs, the instantaneous flow rates (indicated by the symbol P(t)) of the blood flowing from the trunk to the extrmities and vice versa, may be derived from the formula

$$P(t) = \frac{dV_b}{dt} = \frac{d(\Delta V_{cw} - \Delta VL)}{dt} .$$

These and other features of the present invention will become more apparent from the following detailed description of an embodiment thereof by way of no limitation in the accompanying drawings, in which:

figure 1 shows a diagrammatic view of the apparatus in figure 1;
figure 2 shows an electric diagram equivalent to the dynamic state of the booth of the plethysmograph in figure 1;
figure 3 shows a view similar to that in figure 1 for a different embodiment of the invention;
figure 4 shows a view similar to that in figure 1 for a further embodiment of the invention;
figure 5 shows a block diagram of a procedure according to the invention.

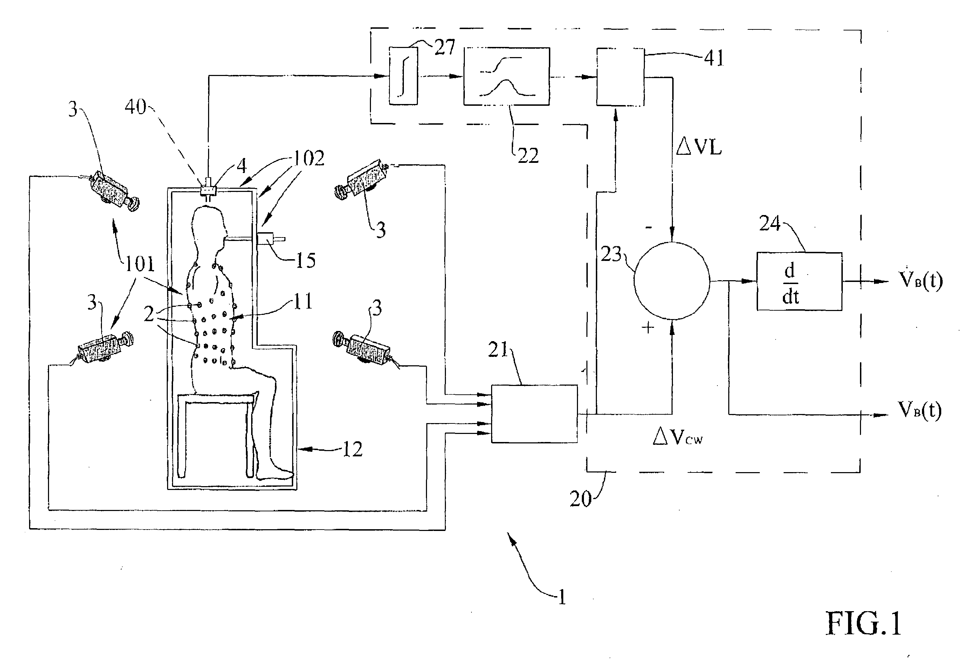

An apparatus I according to the invention, diagrammatically shown in figure 1, is based on the following devices:

- a thoraco-abdominal plethysmograph 101, in this case consisting of an opto-electronic plethysmograph, provided with digital video cameras 3 and markers 2;
- a whole body plethysmograph 102, by way of example of the flow-type, provided with a booth 12 with a mouthpiece 15, openings 4 and a flow sensor 40;
- a microprocessor processing system 20.

The whole body plethysmograph 102 is structured as follows. The booth 12 has a volume of 600 $dm^3$ and is adapted to allow the seating of a subject 11 whose blood flow dynamics between the trunk and the extremities is to be studied. The booth 12 comprises non-transparent parts and transparent parts. Approximately at the level of the mouth of the subject 11, a mouthpiece 15 allows the passage of air from the outside to the inside.

On the upper wall of the booth 12, openings 4 allow the passage of air between the outside and the inside of said booth 12; a traditional flow sensor 40 with a pneumotachograph (provided with Lilly or Silverman or Fleisch resistors) to measure the air flow passing through the openings 4 is applied to each of said openings 4.

The opto-electronic thoraco-abdominal plethysmograph 101 is structured as follows. In front of the booth 12, video cameras 3 are arranged so as to film inside the booth. Such video cameras 3 are connected with an output to an electronic detection device 21.

A plurality of markers 2, consisting of small plastic half-spheres covered by reflective material is applied to the subject 11 at preset positions on the surface of the trunk. The application of such markers 2 is performed by means of the application of double-sided hypoallergenic paper tape.

Said electronic detection device 21 is an electronic system which provides the value of the volume variation of the trunk ($\Delta V_{cx}$) on the outside on the basis of processing performed on the digital images obtained from said video cameras 3 by means of the tracing of the positions in space of the markers 2.

The microprocessor system 20 comprises an integrating circuit 27, an anti-filter 41, an operation block 23 and a differentiator circuit 24.

The flow sensor 40 provides the value of the gas flow in and out of the lungs. Such a value is integrated by the integrating circuit 27 in order to obtain the changes in gas volume of the lungs.

Such a value is anti-filtered by the anti-filter 22 (the explanation will be carried out hereafter).

Downstream of the anti-filter 22 there is a clamping circuit 41. The reason for the use of such a clamping circuit 41 will be explained hereafter.

The values of the outputs of the electronic detection device 21 and clamping circuit 41 are subtracted in the operation block 23 in order to obtain the value of the amount of blood flowing between the trunk and the extremities ($V_b$) and is provided on the outside.

Finally, such a value $V_b$ is provided on the outside, and also differentiated by the differentiator circuit 24 to compute the flow rate *P(t)* thereof.

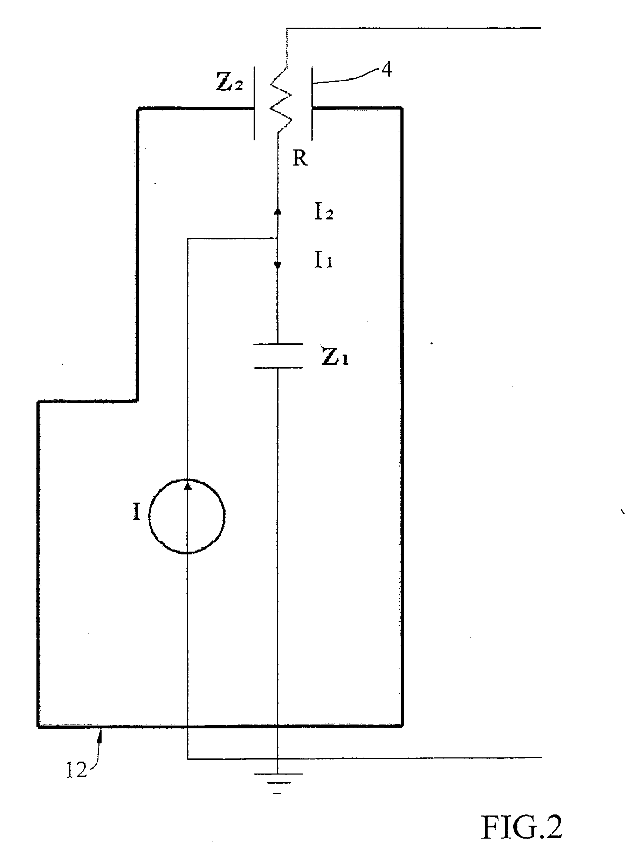

The reason for the use of the anti-filter 22 is explained hereafter. The dynamical phenomena occurring within the booth 12 must indeed be taken into account. For this purpose, it is helpful to refer to an equivalent circuit shown in figure 2, in which:

- a current generator (I) represents the variations of lung volume generated by the subject under examination, which partially causes air flow through the walls of the booth and partially compresses the volume of gas contained in the booth during inspiration and decompresses the gas during expiration,

- a capacitance C represents the compressibility of the gas contained in the booth and its value depends on the air volume surrounding the subject 11 (equivalent to the volume of the booth subtracted of the total body volume of the subject). An impedance

$$Z_1 = \frac{1}{sC}$$ is associated to the capacitance C,

- a resistance R (and impedance $Z_2 = R$; such a resistance is valid when the above said Lilly or Silverman resistors are used; when the above said Fleisch resistors are used, the inductance must also be included) represents the pneumotachograph 4;

- a current $I_2$ represents the gas flow through the pneumotachograph 4, and results being correlated to I by the following transfer function: $I_2 = \dfrac{1}{1 + sRC} I$, which is the equation of a low pass filter having a cut-off frequency $f_b$, equivalent to $f_b = \dfrac{1}{2\pi RC}$

To correct the attenuation and the phase shift introduced by the booth dynamics it is therefore necessary to apply an appropriate anti-filter consisting of a filter, the transfer function of which is:

$$H(s) = \frac{1 + s \cdot \tau_{fb}}{1 + s \cdot \tau_{fh}}$$

where

$$\tau_{fb} = \frac{1}{2\pi \cdot f_b}$$

and

$$\tau_{fh} = \frac{1}{2\pi \cdot f_h}$$

with cut-off frequency $f_h$ at high frequencies being for instance 20 Hz.

The reason for the use of the clamping circuit 41 is now explained. Within the booth 12, the subject 11 produces heat giving rise to a thermal drift which must be corrected by moans of high pass filtering of the signal, which however may be difficult to define. A correction of the drift is therefore obtained by aligning the $\Delta V_{cw}$ plots (which are not subject to drift) from the output of the electronic detection device 21 with the output of the anti-filter 22 at the end of the inspiration (when there is usually no blood flow and therefore the two plots coincide) breath by breath and by linearly correcting the signal $\Delta$VL in the segment between two points of zero flow at the beginning and end of the inspirations. Such a function is performed by the clamping circuit 41.

There may be variants to the embodiment of the present invention.

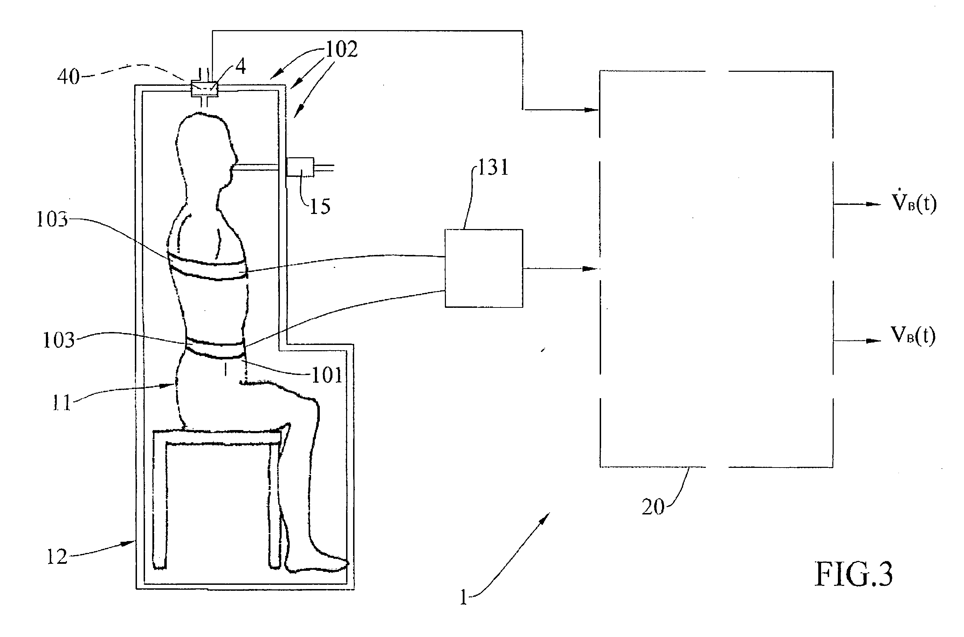

For instance, as shown in figure 3, the thoraco-abdominal plethysmograph 101 could be formed by an above mentioned respiratory inductance plethysmograph (RIP) instead of an opto-electronic plethysmograph. It should be noted that the above mentioned elastic bands 103 comprising electrical coils are used instead of the video cameras 3. The apparatus uses an electronic detection device 131 allowing measurement of the self inductance of said electric coils, providing the value of the volute changes of the trunk to the microprocessor system 20.

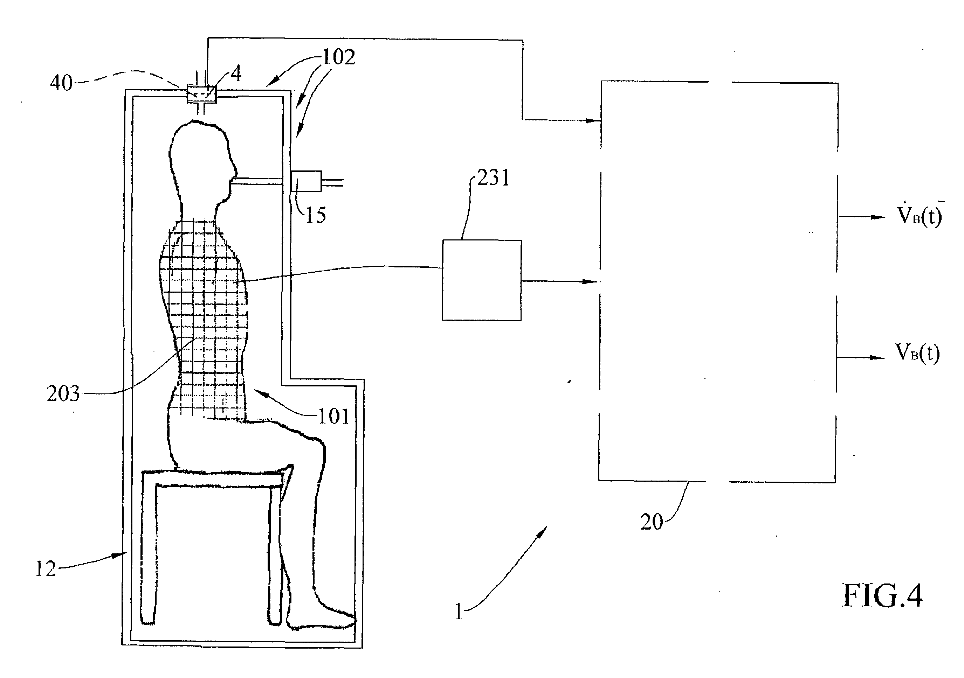

According to a further variant, shown in figure 4, the thoraco-abdominal plethysmograph 101 could be formed by the aforementioned thoraco-abdominal plethysmography contained in a shirt 203; the output is read by an electronic detection device 231. For instance, a product designated as Respitrace produced by Vivometrics could be used.

In the two variants just described above, the booth 12 does not need to be transparent, as the use of video cameras is not necessary.

Variants may also be used for the whole body plethysmograph 102, by using, for instance, a volume displacement plethysmograph instead of a variable flow plethysmograph.

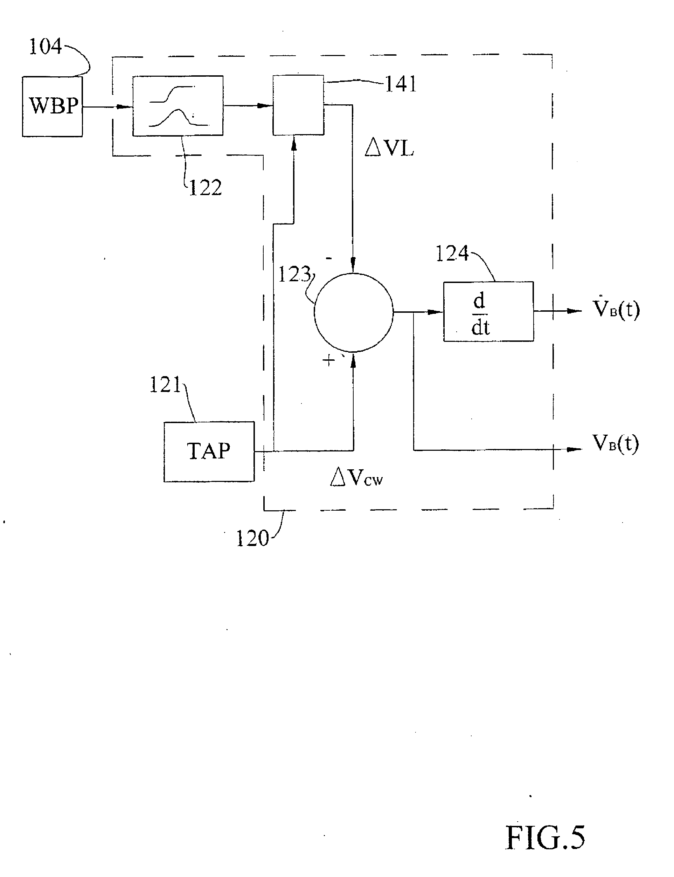

Figure 5 shows a process according to the invention, comprising:

- a measurement 104 of the variations of gas volume of the lungs ($\Delta$VL) by means of a whole body plethysmograph..

- a measurement 121 of the volume variations of the trunk ($\Delta V_{cw}$) by means of thoraco-abdominal plethysmography; such a measurement 121 is concurrent with the above said measurement 104; said measurement 121 is performed by means of a thoraco-abdominal plethysmograph (which may be, without distinction, an opto-electronic plethysmograph, a respiratory inductance plethysmograph or a plethysmograph contained in clothing,);

- a subsequent processing 120 of the volume variations of the trunk ($\Delta V_{cw}$) and of the variations of gas volume of the lungs in order to measure the blood flow between the thoraco-abdominal compartment and the extremities ($V_b$).

The latter processing 120 comprises:

- an anti-filtering 122 of the variations of gas volume of the lungs ($\Delta$VL);

- a drift correction 141 downstream of the anti-filtering 122;
- a computation 123 of the difference between the volume variations of the trunk ($\Delta V_{cw}$) and the volume variations of the lungs ($\Delta VL$) to obtain the volume of blood shifted between the trunk and the extremities ($V_b$);

differentiation 124 of $V_b$ to obtain the flow of blood *P(t)* between the trunk and the extremities

## Claims

1. An apparatus (1) for measuring the blood flow between the trunk and the extremities ($V_b$), comprising a whole body plethysmograph (102; 4, 12, 15, 22, 40) for measuring the variations of gas volume of the lungs ($\Delta VL$), a thoraco-abdominal plethysmograph (101; 2, 3, 21, 103, 203) for measuring the volume variations of the trunk ($\Delta V_{cw}$) and processing system (20, 21, 22, 23, 24, 41, 131, 231) to measure said variations of gas volume of the lungs ($\Delta VL$) with said volume variations of trunk ($\Delta V_{cw}$) so as to compute the blood shifts between the trunk and the extremities ($V_b$), said processing system comprising electronic detection devices (21, 131, 231) adapted to measure said volume variations of the trunk ($\Delta V_{cw}$) and a clamping circuit (41) adapted to align the plot of said variations of gas volume of the lungs ($\Delta VL$) with the plot of said volume variations of the trunk ($\Delta V_{cw}$) at the end of the inspiration breath by breath and by linearly correcting the variations of gas volume of the lungs ($\Delta VL$) in the segment between two points of zero flow at the beginning and end of the inspirations.

2. An apparatus according to claim 1, **characterised by** the said thoraco-abdominal plethysmograph (101) is an opto-electronic plethysmograph comprising digital video cameras (3) adapted to film reflective markers (2) placed on the chest and abdomen of a subject being examined (11) seated in a transparent booth (12), with an electronic detection device (21) being provided as connected with an input to said digital video cameras (3), said electronic detection device (21) allowing measurement of the volume variation of the trunk ($\Delta V_{cx}$) on the basis of processing performed on the digital images obtained from said video cameras (3) by means of the tracing of the positions in space of said markers.

3. An Apparatus according to claim 1, in which the said thoraco-abdominal plethysmograph (101) is a respiratory inductance plethysmograph computing elastic bands (103) provided with wires, with an electronic detection device (131) being provided, allowing the measurement of the volume variation of the trunk ($\Delta V_{cx}$) on the basis of electric parameters of said wires.

4. An apparatus according to claim 1, in which the said thoraco-abdominal plethysmograph (101) is a respiratory inductance plethysmograph contained in a shirt (203), with an electronic detection device (231) being provided, allowing the measurement of the volume variation of the trunk ($\Delta V_{cx}$).

5. An apparatus according to any of the preceding claims, **characterised by** the said whole body variable flow plethysmograph (102) comprising a booth (12) provided with a mouthpiece (15) and openings (4) comprising flow sensors (40).

6. An apparatus according to any of claims 1-4, **characterised by** said volume displacement whole body plethysmograph (102).

7. An apparatus according to any of the preceding claims, **characterised by** an anti-filter (22) with a transfer function $H(s) = \dfrac{1 + s \cdot \tau_{fb}}{1 + s \cdot \tau_{fh}}$ wherein

$$\tau_{fb} = \frac{1}{2\pi \cdot f_b} \text{ and } \tau_{fh} = \frac{1}{2\pi \cdot f_h} \text{ with } fb \text{ and}$$

*fh* cut-off frequencies at low and high frequencies.

8. An apparatus according to any of the preceding claims, **characterised by** a differentiator circuit (24) allowing to compute the blood flow between the thoraco-abdominal compartment and the periphery (*P (t)*).

9. A process for measuring the blood flow between the trunk and the extremities ($V_b$), comprising a measurement (121) of the volume variation of the trunk ($\Delta V_{cw}$) by means of a thoraco-abdominal plethysmography, a measurement (104) of the variations of gas volume of the lungs ($\Delta VL$) by means of whole body plethysmography, and a processing (120) of said volume variations of the trunk ($\Delta V_{ow}$) and of said volume variations of the lungs ($\Delta VL$) so as to measure the volume of blood shifted between the trunk and the extremities ($V_b$), said measurements (10, 121) being concurrent, said processing comprising the alignment of the plot of said variations of gas volume of the lungs ($\Delta VL$) with the plot of said volume variations of the trunk ($\Delta V_{cw}$) at the end of the inspiration breath by breath and the linearly correction of the variations of gas volume of the lungs ($\Delta VL$) in the segment between two points of zero flow at the beginning and end of the inspirations.

10. A process according to claim 9, **characterised by** an anti-filtering (122) of said gas volume value in the thoraco-abdominal compartment (ΔVL) by means of an anti-filter (22) with a transfer function

$$H(s) = \frac{1 + s \cdot \tau_{fb}}{1 + s \cdot \tau_{fh}} \quad \text{wherein} \quad \tau_{fb} = \frac{1}{2\pi \cdot f_b}$$

and $\tau_{fh} = \frac{1}{2\pi \cdot f_h}$ with *fb* and *fh* cut-off frequencies at low and high frequencies.

11. A process according to any of claims 9-10, **characterised by** differentiation (124) of the volume of blood shifted between the trunk and the extremities ($V_b$) allowing computation of the flow rate (*P(t)*) of blood between the thoraco-abdominal compartment and the periphery.

**Patentansprüche**

1. Vorrichtung (1) zum Messen des Blutflusses zwischen dem Rumpf und den Extremitäten ($V_b$), die einen Ganzkörper-Plethysmographen (102; 4, 12, 15, 22, 40) zum Messen der Änderungen eines Gasvolumens der Lungen (ΔVL) aufweist, einen Thorakoabdominal-Plethysmographen (101; 2, 3, 21, 103, 203) zum Messen der Volumenänderungen des Rumpfs ($\Delta V_{cw}$) und ein Verarbeitungssystem (20, 21, 22, 23, 24, 41, 131, 231), um die Änderungen eines Gasvolumens der Lungen (ΔVL) mit den Volumenänderungen des Rumpfs ($\Delta V_{cw}$) zu messen, um die Blutverschiebungen zwischen dem Rumpf und den Extremitäten ($V_b$) zu berechnen, wobei das Verarbeitungssystem elektronische Detektionsvorrichtungen (21, 131, 231) aufweist, die dazu geeignet sind, die Volumenänderungen des Rumpfs ($\Delta V_{cw}$) zu messen, und eine Klemmschaltung (41), die dazu geeignet ist, die grafische Darstellung der Änderungen eines Gasvolumens der Lungen (ΔVL) mit der grafischen Darstellung der Volumenänderungen des Rumpfs ($\Delta V_{cw}$) am Ende der Einatmung Atemzug für Atemzug auszurichten und eine lineares Korrigieren der Änderungen eines Gasvolumens der Lungen (ΔVL) in dem Abschnitt zwischen zwei Punkten von keinem Fluss am Anfang und am Ende der Einatmungen vorzunehmen.

2. Vorrichtung nach Anspruch 1, **dadurch gekennzeichnet, dass** der Thorakoabdominal-Plethysmograph (101) ein optoelektronischer Plethysmograph ist, der digitale Videokameras (3) aufweist, die dazu geeignet sind, Reflexionsmarkierer (2), die auf der Brust und dem Bauch eines in eine transparente Kabine (12) gesetzten bzw. gelegten Subjekts, das untersucht wird, platziert sind, zu filmen, wobei eine elektronische Detektionsvorrichtung (21) derart vorgesehen ist, dass sie mit einem Eingang zu den digitalen Videokameras (3) verbunden ist, wobei die elektronische Detektionsvorrichtung (21) mittels des Verfolgens der Positionen der Markierer im Raum eine Messung der Volumenänderung des Rumpfs ($\Delta V_{cx}$) auf der Basis einer Verarbeitung zulässt, die an den von den Videokameras (3) erhaltenen digitalen Bildern durchgeführt wird.

3. Vorrichtung nach Anspruch 1, wobei der Thorakoabdominal-Plethysmograph (101) ein respiratorischer Induktanz-Plethysmograph ist, der elastische Bänder (103) aufweist, die mit Drähten versehen sind, wobei eine elektronische Detektionsvorrichtung (131) vorgesehen ist, die die Messung der Volumenänderung des Rumpfs ($\Delta V_{cx}$) auf der Basis elektrischer Parameter der Drähte zulässt.

4. Vorrichtung nach Anspruch 1, wobei der Thorakoabdominal-Plethysmograph (101) ein respiratorischer Induktanz-Plethysmograph ist, der ein einem Hemd (203) enthalten ist, wobei eine elektronische Detektionsvorrichtung (231) vorgesehen ist, die die Messung der Volumenänderung des Rumpfs ($\Delta V_{cx}$) zulässt.

5. Vorrichtung nach einem der vorangehenden Ansprüche, **dadurch gekennzeichnet, dass** der Ganzkörper-Plethysmograph für variablen Fluss (102) eine Kabine (12) aufweist, die mit einem Mundstück (15) und Öffnungen (4) mit Durchflusssensoren (40) versehen ist.

6. Vorrichtung nach einer der Ansprüche 1 - 4, **gekennzeichnet durch** einen Volumenversatz-Ganzkörper-Plethysmographen (102).

7. Vorrichtung nach einem der vorangehenden Ansprüche, **gekennzeichnet durch** ein Anti-Filier (22) mit einer Übertragungsfunktion

$$H(s) = \frac{1 + s \cdot \tau_{f_b}}{1 + s \cdot \tau_{f_h}}, \quad \text{wobei} \quad \tau_{f_b} = \frac{1}{2\pi \cdot f_b} \quad \text{und}$$

$$\tau_{f_h} = \frac{1}{2\pi \cdot f_h}, \quad \text{wobei } f_b \text{ und } f_h \text{ Grenzfrequenzen}$$

bei niedrigen und hohen Frequenzen sind.

8. Vorrichtung nach einem der vorangehenden Ansprüche, **gekennzeichnet durch** eine Differenzierschaltung (24), die zulässt, den Blutfluss zwischen dem Thorakoabdominalbereich und der Umgebung zu berechnen (P(t)).

9. Verfahren zum Messen des Blutflusses zwischen dem Rumpf und den Extremitäten ($V_b$), das eine Messung (121) der Volumenänderungen des Rumpfs ($\Delta V_{cw}$) mittels einer Thorakoabdominal-Plethysmographie aufweist, eine Messung (104) der Änderungen eines Gasvolumens der Lungen ($\Delta VL$) mittels einer Ganzkörper-Plethysmographie und eine Verarbeitung (102) der Volumenänderungen des Rumpfs ($\Delta V_{cw}$) und der Volumenänderungen des Lungen ($\Delta VL$), um das Volumen von zwischen dem Rumpf und den Extremitäten ($V_b$) verschobenem Blut zu messen, wobei die Messungen (10, 121) gleichzeitig erfolgen, wobei die Verarbeitung die Ausrichtung der grafischen Darstellung der Änderungen eines Gasvolumens der Lungen ($\Delta VL$) mit der grafischen Darstellung der Volumenänderungen des Rumpfs ($\Delta V_{cw}$) am Ende der Einatmung Atemzug für Atemzug und die lineare Korrektur der Änderungen eines Gasvolumens der Lungen ($\Delta VL$) in dem Abschnitt zwischen zwei Punkten von keinem Fluss am Anfang und am Ende der Einatmungen aufweist.

10. Verfahren nach Anspruch 9, **gekennzeichnet durch** eine Anti-Filterung (122) des Gasvolumenwerts in dem Thorakoabdominalbereich ($\Delta VL$) mittels eines Anti-Filters (22) mit einer Übertragungs-

funktion $H(s) = \dfrac{1 + s \cdot \tau_{f_b}}{1 + s \cdot \tau_{f_h}}$ , wobei

$\tau_{f_b} = \dfrac{1}{2\pi \cdot f_b}$ und $\tau_{f_h} = \dfrac{1}{2\pi \cdot f_h}$ , wobei $f_b$ und

$f_h$ Grenzfrequenzen bei niedrigen und hohen Frequenzen sind.

11. Verfahren nach einem der Ansprüche 9-10, **gekennzeichnet durch** eine Differenzierung (124) des Volumens von zwischen dem Rumpf und den Extremitäten ($V_b$) verschobenem Blut, welche eine Berechnung der Flussrate (P(t)) von Blut zwischen dem Thorakoabdominalbereich und der Umgebung zulässt.


## Revendications

1. Appareil (1) pour mesurer le flux sanguin entre le tronc et les extrémités ($V_b$), comprenant un pléthysmographe pour corps entier (102 ; 4, 12, 15, 22, 40) pour mesurer les variations de volume gazeux des poumons ($\Delta VL$), un pléthysmographe thoraco-abdominal (101 ; 2, 3, 21, 103, 203) pour mesurer les variations de volume du tronc ($\Delta V_{cw}$) et un système de traitement (20, 21, 22, 23, 24, 41, 131, 231) pour mesurer lesdites variations de volume gazeux des poumons ($\Delta VL$) avec lesdites variations de volume du tronc ($\Delta V_{cw}$) afin de calculer les décalages sanguins entre le tronc et les extrémités ($V_b$), ledit système de traitement comprenant des dispositifs de détection électroniques (21, 131, 231) adaptés pour mesurer lesdites variations de volume du tronc ($\Delta V_{cw}$) et un circuit de fixation de niveau (41) adapté pour aligner le diagramme desdites variations de volume gazeux des poumons ($\Delta VL$) avec le diagramme desdites variations de volume du tronc ($\Delta V_{cw}$) à la fin de l'inspiration souffle par souffle et en corrigeant linéairement les variations de volume gazeux des poumons ($\Delta VL$) dans le segment situé entre deux points de flux nul au début et à la fin des inspirations.

2. Appareil selon la revendication 1, **caractérisé en ce que** ledit pléthysmographe thoraco-abdominal (101) est un pléthysmographe opto-électronique comprenant des caméras vidéo numériques (3) adaptées pour filmer des marqueurs réfléchissants (2) placés sur la poitrine et l'abdomen d'un sujet à examiner (11) assis dans une cabine transparente (12), un dispositif de détection électronique (21) étant prévu, connecté par une entrée auxdites caméras vidéo numériques (3), ledit dispositif de détection électronique (21) permettant la mesure de la variation de volume du tronc ($\Delta V_{cx}$) en se basant sur le traitement effectué sur les images numériques obtenues par lesdites caméras vidéo (3) au moyen du tracé des positions desdits marqueurs dans l'espace.

3. Appareil selon la revendication 1, dans lequel ledit pléthysmographe thoraco-abdominal (101) est un pléthysmographe à inductance respiratoire comprenant des bandes élastiques (103) pourvues de fils métalliques, un dispositif de détection électronique (131) étant prévu, permettant la mesure de la variation de volume du tronc ($\Delta V_{cx}$) en se basant sur les paramètres électriques desdits fils.

4. Appareil selon la revendication 1, dans lequel ledit pléthysmographe thoraco-abdominal (101) est un pléthysmographe à inductance respiratoire contenu dans une chemise (203), un dispositif de détection électronique (231) étant prévu, permettant la mesure de la variation de volume du tronc ($\Delta V_{cx}$),

5. Appareil selon l'une quelconque des revendications précédentes, **caractérisé en ce que** ledit pléthysmographe à flux variable pour corps entier (102) comprend une cabine (12) pourvue d'une embouchure (15) et d'ouvertures (4) comprenant des capteurs de flux (40).

6. Appareil selon l'une quelconque des revendications 1 à 4, **caractérisé par** ledit pléthysmographe à déplacement de volume pour corps entier (102).

7. Appareil selon l'une quelconque des revendications précédentes, **caractérisé par** un antifiltre (22) ayant une fonction de transfert $H(s) = \dfrac{1 + s \cdot \tau_{f_b}}{1 + s \cdot \tau_{f_h}}$, où

$\tau_{f_b} = \dfrac{1}{2\pi \cdot f_b}$ et $\tau_{f_h} = \dfrac{1}{2\pi \cdot f_h}$, fb et fh étant les fréquences de coupure aux basses et hautes fréquences.

8. Appareil selon l'une quelconque des revendications précédentes, **caractérisé par** un circuit différenciateur (24) permettant de calculer le flux sanguin entre le compartiment thoraco-abdominal et la périphérie (P(t)).

9. Procédé pour mesurer mesurer le flux sanguin entre le tronc et les extrémités ($V_b$), comprenant une mesure (121) des variations de volume du tronc ($\Delta V_{cw}$) au moyen d'une pléthysmographie thoraco-abdominale, une mesure (104) des variations de volume gazeux des poumons ($\Delta VL$) au moyen d'une pléthysmographie du corps entier, et un traitement (120) desdites variations de volume du tronc ($\Delta V_{cw}$) et desdites variations de volume des poumons ($\Delta VL$) afin de mesurer le volume de sang décalé entre le tronc et les extrémités ($V_b$), lesdites mesures (10, 121) étant simultanées, ledit traitement comprenant l'alignement du diagramme desdites variations de volume gazeux des poumons ($\Delta VL$) avec le diagramme desdites variations de volume du tronc ($\Delta V_{cw}$) à la fin de l'inspiration souffle par souffle et la correction linéaire des variations de volume gazeux des poumons ($\Delta VL$) dans le segment situé entre deux points de flux nul au début et à la fin des inspirations.

10. Procédé selon la revendication 9, **caractérisé par** un antifiltrage (122) de ladite valeur de volume gazeux dans le compartiment thoraco-abdominal ($\Delta VL$) au moyen d'un antifiltre (22) ayant une fonction

de transfert $H(s) = \dfrac{1 + s \cdot \tau_{f_b}}{1 + s \cdot \tau_{f_h}}$, où

$\tau_{f_b} = \dfrac{1}{2\pi \cdot f_b}$ et $\tau_{f_h} = \dfrac{1}{2\pi \cdot f_h}$, fb et fh étant les fréquences de coupure aux basses et hautes fréquences.

11. Procédé selon l'une quelconque des revendications 9 à 10, **caractérisé par** la différenciation (124) du volume de sang décalé entre le tronc et les extrémités ($V_b$) permettant le calcul du débit (P(t)) de sang entre le compartiment thoraco-abdominal et la périphérie.

FIG.1

EP 2 051 632 B1

FIG.2

FIG.3

$\dot{V}_B(t)$

$V_B(t)$

FIG.4

EP 2 051 632 B1

FIG.5

14

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 3511237 A
- IT MI001188 A1
- US 5331968 A
- US 3731184 A
- US 4308872 A
- US 4373534 A
- US 4433693 A
- US 4452252 A
- US 4494553 A
- US 4807640 A
- US 4815473 A
- US 4817625 A
- US 4834109 A
- GB 2116725 A

### Non-patent literature cited in the description

- **Aliverti A. ; Macklem P.T.** How and why exercise is impaired in COPD. *Respiration,* 2001, vol. 68 (229), 239
- **Aliverti A. ; R. L. Dellacà ; P. Lotti ; S. Bertini ; R. Duranti ; G. Scano ; J. Heyman ; A. Lo Mauro ; A. Pedotti ; P. T. Macklem.** Influence of expiratory flow-limitation during exercise on systemic oxygen delivery in humans. *European Journal of Applied Physiology,* 2005
- **Duke G. J.** Cardiovascular effects of mechanical ventilation. *Critical Care and Resuscitation,* 1999, vol. 1, 388-399
- **H. Fessler ; S. Permutt.** Interaction between the circulatory and ventilatory pumps. *Thorax,* 1995, vol. 57
- **Iandelli I ; Aliverti A ; Kayser B ; Dellacà R ; Cala SJ ; Duranti R ; Kelly S ; Scano G ; Sliwinski P ; Yan S.** Determinants of exercise performance in normal men with externally imposed expiratory flow limitation. *J Appl Physiol,* 2002, vol. 92, 1943-1952
- **Lloyd. C.** Mechanical cardiopulmonary interdependence. *J. Appl. Physiol.: Respirat. Environ,* 1982, vol. 52, 333-339
- **Lu, K. ; J.W. Clark, Jr. ; F.H. Ghorbel ; D.L. Ware ; A. Bidani.** A human cardiopulmonary system model applied to the analysis of the Valsalva maneuver. *Am J Physiol Heart Circ Physiol,* 2001, vol. 281, H2661-H2679
- **Miller J.D. ; Pegelow D.F. ; Jacques A. J. ; Dempsey J.** Effects of augmented respiratory muscle pressure production on locomotor limbs venous return during calf contraction exercise. *J Appl Physiol,* 2005
- Mechanical interaction of respiration and circulation. **Permutt S. ; A. Wise.** Handbook of physiology: the respiratory system. 1986, vol. III, 36
- **Ranieri V.M. ; M. Dambrosio ; N. Brienza.** Intrinsic PEEP and cardiopulmonary interaction in patients with COPD and acute ventilatory failure. *Eur Respir J,* 1996, vol. 9, 1283-1292
- **Scharf, S.M. ; J.A. Bianco ; D.E. Tow ; R. Brown.** The effects of large negative intrathoracic pressure on left ventricular function in patients with coronary artery disease. *Circulation,* 1981, vol. 63, 871-875
- **Scharf, S.M. ; R. Brown ; N. Saunders ; L.H. Green.** Effects of normal and loaded spontaneous inspiration on cardiovascular function. *J. Appl. Physiol.: Respirat. Environ. Exercise Physiol.,* 1979, vol. 47, 582-590
- **Takata, M. ; Wise, R. A. ; Robotham, J. L.** Effects of abdominal pressure on venous return: abdominal vascular zone conditions. *J. Appl. Physiol.,* 1990, vol. 69 (6), 1961-1972
- **Mead J.** Volume displacement body plethysmograph for respiratory measurements in human subjects. *J Appl Physiol,* 1960, vol. 15, 736-740
- **Ferrigno G ; Carnevali P ; Aliverti A ; Molteni F ; Beulke G ; Pedotti A.** Three-dimensional Optical Analysis of Chest Wall Motion. *J Appl Physiol,* 1994, vol. 77 (3), 1224-1231
- **Cala SJ ; Kenyon C ; Ferrigno G ; Carnevali P ; Aliverti A ; Pedotti A ; Macklem PT ; Rochester DF.** Chest wall and lung volume estimation by optical reflectance motion analysis. *J Appl Physiol,* 1996, vol. 81 (6), 2680-2689
- **Carnevali P ; Ferrigno G ; Aliverti A ; Pedotti A.** A new method for 3D optical analysis of chest wall motion. *Technology and Health Care,* 1996, vol. 4, 43-65
- **Aliverti A ; R. Dellacà ; P. Pelosi ; D. Chiumello ; A. Pedotti ; L. Gattinoni.** Opto-electronic plethysmography in intensive care patients. *Am. J. Respir. Crit. Care Med.,* 2000, vol. 161, 1546-1552

- **Aliverti A ; R. Dellacà ; P. Pelosi ; D. Chiumello ; L. Gattinoni ; A. Pedotti.** Compartmental analysis of breathing in the supine and prone positions by Opto-Electronic Plethysmography. *Ann Biomed Eng,* 2001, vol. 29, 60-70
- **Aliverti A ; Pedotti A.** Opto-electronic Plethysmography. *Monaldi Arch Chest Dis,* 2003, vol. 59 (1), 12-6
- **E. R. Post ; M. Orth.** Smart Fabric, or Washable Computing. *Proceedings of First International Symposium on Wearable Computers,* 1997, 167-168
- **Della Santa A ; Mazzoldi A ; De Rossi D.** Dressware: wearable hardware. *Material Sci Eng,* 1999, (C7), 31-37
- a New Textile-Based Wearable Device for Biological Signal Monitoring. Applicability in Daily Life and Clinical Setting. **Di Rienzo M ; Rizzo F ; Parati G ; Brambilla G ; Ferratini M ; Castiglioni P.** Proc. IEEE EMB Conference 2005. IEEE Press, 2005